# EUROPEAN PATENT APPLICATION

(11) **EP 0 935 965 A1**
(43) Date of publication of application: **18.08.1999**
(21) Application number: 97919659.9
(22) Date of filing: 23.04.1997
(51) Int. Cl.: A61K 38/16, A61K 35/28, A01N 63/02, A01N 37/18, C07K 14/47

(54) **ANTIPYLORI AGENT**

(30) Priority: 23.04.1996 JP 10168196
(71) Applicant: TORAY INDUSTRIES, INC., Tokyo 103-8666 (JP)
(72) Inventor: KOUGO, Yutaka, Asahikawa-shi, Hokkaidou 078 (JP); ONO, Minoru, Asahikawa-shi, Hokkaidou 071-01 (JP); NAGAI, Kouzou, Tokorozawa-shi, Saitama 359 (JP); MORIYAMA, Masami, Yokohama-shi, Kanagawa 247 (JP); SAITOU, Makiko, Musashino-shi, Tokyo 180 (JP)
(74) Representative: Kador & Partner
(86) International application number: JP9701409
(87) International publication number: WO9739764

(57) **Abstract**

It was made apparent that an antimicrobial peptide has an effective sterilizing and bacteria eliminating function for pylori, i.e., Helicobacter pylori (H. pylori). On the basis of this function, it was found that an antimicrobial peptide is effective as an antiulcer agent and a wound healing agent.

## Description

### Technical Field

The present invention relates to an anti Helicobacter pylori material, and particularly to a therapeutic agent for removing pylori which parasitizes the human stomach, preventing infection therewith, and healing ulcers and wounds.

### Background Art

Pylori, i.e., Helicobacter pylori (abbreviated to "H. pylori" hereinafter) represents bacteria isolated from the mucosae of gastric ulcer patients by Marshall and Warrent in Australia, and considered as the main causal bacteria of chronic gastritis, and the strongest adverse factor which influences healing and relapse of a peptic ulcer and diseases of the upper digestive tract, such as gastric cancer lymphoma or the like. Therefore, the correlation between H. pylori and these diseases increasingly attracts attention. For example, statistical immunological examination reveals that H. pylori is detected in 65% of gastritis patients, 86% of gastric ulcer patients, and about 10% of duodenal ulcer patients, while only about 30% of humans of health and the same ages as the patients are infected with H. pylori. Various experiments also indicate that in the case of chronic gastritis accompanied with infection with H. pylori, the risk of occurrence of a peptic ulcer within 10 to 20 years from infection reaches 3 to 12 times. H. pylori has the relation to the relapse of an ulcer which has been cured, and gastric ulcer patients infected with H. pylori have the relapse of an ulcer at a rate of as high as 70% or more within 1 year, while patients who are not infected with H. pylori have the relapse at a rate of only about 10%. For example, it is said that of patients of ulcers cured with a histamine H2 receptor antagonist (H2 blocker) and infected with H. pylori, 50% of patients have the relapse of an ulcer within 6 months, and 95% of patients have the relapse of an ulcer within 2 years (for example, Nikkei Science, pp. 54-61, April 1996).

At present, the relation between infection with H. pylori and occurrence and recurrence of chronic gastritis and peptic ulcer substantially becomes an established theory. If H. pylori can completely be removed, inflammation substantially completely disappears, but reinfection with H. pylori causes recurrence of gastritis. It is thus found that infection with H. pylori actually causes chronic superficial gastritis in every person. Therefore, removal of H. pyroli constantly present in the gastric mucosal layer not only prevents occurrence of a peptic ulcer but also results in prevention of recurrence of an ulcer. In "NIH Consensus Conference" held in the United States of America in February, 1994, a united opinion relating to "Helicobacter pylori in peptic ulcer disease" was published, and it was recommended that "a peptic ulcer accompanied with infection with H. pylori requires sterilization therapy using an antisecretory agent and an antimicrobial agent regardless of initial occurrence or recurrence".

H. pylori represents gram negative helical or S-shaped bacteria of 0.5 to 1.0 x 2.5 to 4.0 m which belong to Helicobacter, and have 4 to 6 polar polytrichous flagella. H. pylori is characterized by fixing and proliferating in the mucosal layer which covers the substantially entire gastric wall, and having strong urease activity. Particularly, it is known that H. pylori lives at a high density in the pylori, and does not live in the gastric mucosa having intestinal metaplasia. Also H. pylori is not detected in the dueodenum, the intestine and esophageal mucosa, but lives at a high frequency in the duodenal mucosa having intestinal metaplasia, and H. pylori is thought to closely relate to recurrence of a duodenal ulcer. In recent years, infection by an endoscope has attracted attention, and there has been demand for sufficient disinfection of the endoscope.

Although H. pylori was classified in Campylobacter to which causal bacteria of diarrhea belong at the beginning of discovery, H. pylori was classified as a new independent genus close to Wolinella rather than Campylobacter by a molecular generic method, e.g., the homology of 16S libosomal RNA gene base arrangement and the composition of fatty acids. Clinical isolates are spiral bacteria grown under microaerophilic conditions in a Skilo medium and have urease positive properties, and are identified as H. pylori. From the gene taxonomic viewpoint, it is confirmed that DNA homology and homology of libosomal RNA gene base arrangement are 70% or more, and H. pylori is a species composed of homogeneous strains.

On the other hand, H. pylori isolates suggest a difference in antigenicity between stains by an immunoblot or ELISA method using the serum of patients, but subspecies and subtypes in the species, such as the human serum protein type and the like, have not been established yet.

As described above, elimination or sterilization of H. pylori constantly present in the gastric mucosal layer results in not only suppression of occurrence of a peptic ulcer but also prevention of recurrence of an ulcer, but there has been no antimicrobial agent individually specifically acting on H. pylori. Therefore, attempts have been made to combine an antimicrobial agent as a main agent and various medicines for eliminating bacteria. In Europe and America, the mainstream is three-agent combined therapy using a combination of a bismuth formulation, metronidazole and antimicrobial agent, or two-agent combined therapy using a combination of a proton pump inhibitor (PPI) as an oxyntic inhibitor and amoxicillin.

However, the three-agent combined therapy can achieve a rate of elimination of as high as 80% or more, but has the fault of a high incidence of an adverse effect, and the two-agent combined therapy has a low incidence of an adverse effect but has the fault of a low rate of elimination of bacteria. In addition, in the three-agent combined therapy, appearance of resistant strains is considered as a problem, and appearance of H. pylori having resistance to metronidazole has already been reported.

As another therapy, new three-agent combined therapy is investigated in which PPI is combined with clarithromycin, and amoxicillin. However, it is reported that in order to achieve a rate of elimination of 90% or more, a dose of at least twice an ordinary dose is required. Although combined therapy using a combination of ecabet sodium, plaunotol, sofalcone and amoxicilline has recently been conducted, the rate of elimination of bacteria is not as high as the three-agent combined therapy using a combination of a bismuth formulation, metronidazole and antimicrobial agent.

The above-described NIH recommendation causes a change in medicinal idea in the gastric and colic region, the probability of a new medicine, and a large change in measure against diseases in consideration of quality of life (QOL), and from this viewpoint, development of a substance having an antimicrobial action as a therapeutic agent is considered as a subject.

At present, it is made apparent that initial occurrence and recurrence of a peptic ulcer can be suppressed by eliminating H. pylori, and thus development of an antimicrobial substance causing no adverse effect and no resistant strain is greatly expected.

### Disclosure of Invention

The present invention relates to an anti Helicobacter pylori material, an antiulcer agent and a wound healing agent containing an antimicrobial peptide as an active component, and a method of eliminating H. pylori and a method of treating a patient having an ulcer or wound.

### Best Mode for Carrying Out the Invention

In the present invention, pylori represents H. pylori bacteria belonging to Helicobacter. Although pylori has previously been considered to belong to Campylobacter, pylori is now considered to belong to Helicobacter. Therefore, of course, pylori of the present invention includes bacteria which have previously been considered to belong to Campylobacter.

Antimicrobial peptides of the present invention include the antimicrobial peptide defined in, for example, Journal of Leukocyte Biology, 58(2), pp. 128-186 (1995), and are not limited as long as they have an antimicrobial action on pylori. However, endogenous antimicrobial peptides, particularly, submucosal endogenous antimicrobial peptides, are preferably used.

An example of such endogenous antimicrobial peptides (abbreviated to "EAMP" hereinafter) is a peptide named Magainin and isolated and purified from the skin of Xenopus by Zaslaff et al. in 1987 (Proc. Natl. Acad. Sci. U. S. A., 84(15), pp. 5449-5458, (1987)), and various similar substances generally known as the EAMP family were discovered in vivo.

Known examples of such endogenous antimicrobial peptides include defensin isolated and purified from granulocytes and neutrophils of mammals, bactenicin isolated and purified from bovine neutrophils, and the like. A known example of antimicrobial peptides of human derivation is a peptide derived from bone marrow cells (Myeloid cells) referred to as "FALL39" (Proc. Natl. Acad. Sci. U. S. A., 92(1), pp. 195-199, (1995)).

An example of antimicrobial peptides present in bacteria is Heparan Sulfate Proteoglycan, abbreviated to "HSPG" hereinafter). It is made apparent that this antimicrobial peptide binds to a growth factor such as b-FGF (Basic-Fibroblast Growth Factor) or the like, laminin fibronectin, extracellular substrates such as collagen or the like, and cytokine to exhibit biological activities respectively peculiar to these substrances.

Minoru Ono, one of the inventors, isolated and purified mRNA of syndecan which is one of HSPG and synducin which is a syndecan inducing factor as a new protein inducting factor from a wound fluid of the swine skin (J. Biochem., 269, 31315-31321, 1994, Proc. Natl. Acad. Sci. U. S. A., 91, 11035-11039, 1994). As a result of investigation of the amino acid sequence of synducin, it was found that the sequence is the same as a swine endogenous antimicrobial peptide referred to as proline arginine rich peptide-39 (abbreviated to "PR-39" hereinafter). For PR-39 which is one of endogenous antimicrobial peptides, screening is impossible because the gene of an antimicrobial substance kills E.coli in a conventional E.coli recombination technique, but retrieval of a human gene library is possible. It was thus found that PR-39 is a basic peptide having activity in the acidic region, and acts in the acidic region through no receptor.

As described above, submucosal endogenous antimicrobial peptides have a short history, and even for peptides having antimicrobial activity, research on the relation to H. pylori barely began to be carried out.

As described above, PR-39 is an antimicrobial peptide having antimicrobial activity in the acidic environment, while H. pylori is constantly present in the gastric mucosa under strong acid conditions. Therefore, PR-39 is the most preferable antimicrobial peptide as a peptide having sterilizing and bacteria eliminating actions effective for H. pylori.

In the present invention, antimicrobial peptides belonging to the cathelin family or defensin family are used. Tables 1 and 2 illustrate antimicrobial peptides belonging to each of the families.

As antimicrobial peptides of the cathelin family, for example, SAC7, Bac 5, FALL39, PR-39, and the like shown in Table 1 are preferable, and as antimicrobial peptides of the defensin family, for example, HNP-1, NP-1, RtNP-1, and the like shown in Table 2 are preferably used.

**Table 1**

| Cathelin family | | |
|---|---|---|
| Species | Name of actimicrobial peptide | Derivation |
| Bovine | Bac7 | neutrophil (bone marrow cell) |
| | Bac5 | neutrophil (bone marrow cell) |
| | Indocydine | neutrophil (bone marrow cell) |
| | Dodecapeptide | neutrophil (bone marrow cell) |
| Swine | PR-39 | neutrophil (bone marrow cell) |
| | | samll intestine |
| | PMAP23 | bone marrow cell |
| | PMAP26 | bone marrow cell |
| | Protegrin | bone marrow cell |
| | Cathelin | neutrophil |
| Human | Fall39 | bone marrow cell |
| Rabbit | CAP18 | bone marrow cell |

**Table 2**

| Defensin family | | |
|---|---|---|
| Species | Name of antimicrobial peptide | Derivation |
| Human | HNP-1 | neutrophil |
| | HNP-2 | neutrophil |
| | HNP-3 | neutrophil |
| | HNP-4 | neutrophil |
| | HNP-5 | neutrophil |
| | HNP-6 | Paneth cell |
| Rat | RtNP-1 | neutrophil |
| | RtNP-2 | neutrophil |
| | RtNP-3 | neutrophil |
| Guinea pig | GNP | unknown |
| | RtNP-4 | unknown |
| Rabbit | NP-1 | neutrophil |
| | NP-2 | neutrophil |
| | NP-3a | neutrophil |
| | NP-3b | neutrophil |
| | NP-4 | neutrophil |
| | NP-5 | neutrophil |
| Mouce | MuCr-1 | Paneth cell |
| | MuCr-1a | Paneth cell |
| | MuCr-2 | Paneth cell |
| Bovine | BNBDs | neutrophil |
| | TAP | Tarcheal epithelial cell |

In the present invention, it was confirmed that the structure of synducin which is a cell growth factor is the same as PR-39 which is swine EAMP present in the granules of the polymorphonuclear leucocytes infiltrated in a wound of the skin, and that PR-39 present in the granules of the polymorphonuclear leucocytes infiltrated in a wound of the skin prevents infection with exogenous agents and promotes curing of a wound due to induction of syndecan.

Also an antibody was formed for a fraction exhibiting an anti Helicaobacter pylori action, and the human gastric mucosa and human fibroblasts were stained to confirm that PR-39 related peptides (abbreviated to "PRPP" hereinafter) similar to PR-39 are present.

It was also found that human PRPP has an antimicrobial action (including an anti H. pylori action) and a cell growth action on swine PR-39 or syndecan.

Therefore, in a preferable mode of the present invention, there is provided an anti Helicobacter pylori material, an antiulcer agent, and a wound healing agent containing an antimicrobial peptide containing PR-39 as an active component. PR-39 includes PRPP that is an equivalent substance. PRPP includes peptides having peptide bonds in a region homogeneous to the peptide bonds of PR-39 in homology retrieval. For example, PRPP includes changed peptides of PR-39 exhibiting the same antimicrobial action as PR-39 in MIC assay described in Example 1.

The anti Helicobacter pylori material used in the present invention can orally be administered in a form containing one of these antimicrobial peptides or a midical composition mixture with a carrier, an excipient, or the like which are generally known and pharmacologically acceptable.

Examples of formulations include a tablet, a pill, a capsule, granules, syrup, an emulsion, a suspension, and the like. However, other formulations can also be used as long as the effect of the present invention can be exhibited. Such formulations are produced by a known method and contain a carrier or an excipient which are generally used in the formulation field. Examples of the carrier or excipient for formulations include lactose, maltose, saccharose, starch, magnesium stearate, and the like.

Although the dose of the anti Helicobacter pylori material of the present invention mainly depends upon diseases, symptoms, and the formulation administered, the dose is generally 0.01 to 1500 mg per adult, and preferably a dose of 0.5 to 750 mg can be administered once or divided into several doses.

### Examples

Although the present invention will be described in detail below with reference to examples, the present invention is not limited to these examples.

### Example 1

### Activity measurement of antimicrobial peptide in ATCC human H. pylori strains

PR-39 was isolated, and each fraction was purified by gel filtration in accordance with the method described in J. Biochem., 269, 31315-31321 (1994) and Proc. Natl. Acad. Sci. U. S. A. 91, 11035-11039 (1994). An antimicrobial spectrum of the thus-obtained PR-39 was examined to confirm by MIC assay that PR-39 exhibits antimicrobial activity to H. pylori. Standard stains of H. pylori were bought from ATCC. H. pylori was cultured at 35°C for 2 days under conditions in that 5% sheep blood was added to soy gar II (BBL). Culture was carried out in an Aero Pauchi Compylo System using a Muller-Hinton medium containing 5% horse serum.

MIC assay was performed in accordance with the method of NCCLS (National Comittee for Clinical Laboratory Standard) (NCCLS Vill anova 1983). The results obtained are shown in Table 3.

Table 3 indicates that PR-39 exhibits antimicrobial action on human H. pylori stains of Amercan Type Culture Collectin (ATCC).

**Table 3**

| Antimicrobial activity of PR-39 to human H. pylori strain | | |
|---|---|---|
| | | MIC (µM) |
| ATCC | 43504 | 6.7 - 13.4 |
| | 43579 | 13.2 |
| | 43629 | 6.7 - 13.4 |
| | 43526 | Not grown |
| E coli. | 25922 | 0.18 to 1.7 |

### Example 2

### Activity measurement of antimicrobial peptide in wild strain (clinical isolate)

PR-39 was isolated by the same method as Example 1, and each fraction was purified by gel filtration. An antimicrobial spectrum of the PR-39 obtained was examined to confirm by MIC assay that it exhibits an antimicrobial action on H. pylori. Wild strains of H. pylori were isolated from ulcer patients and cultured. H. pylori was cultured at 35°C for 2 days under conditions in that 5% sheep blood was added to soy gar II (BBL). Culture was carried out in an Aero Pauchi Compylo System using a Muller-Hinton medium containing 5% horse serum.

MIC assay was performed in accordance with the method of NCCLS (National Comittee for Clinical Laboratory Standard) (NCCLS Vill anova 1983). The results obtained are shown in Table 4.

Experiment indicates that PR-39 exhibits a good antimicrobial action on wild stains (clinical isolates) of H. pylori.

**Table 4**

| Antimicrobial activity of PR-39 to H. pylori wild strains | | | |
|---|---|---|---|
| No. | MIC (µM) | No. | MIC (µM) |
| Wild strain 1 | 13.33 | Wild strain 7 | 6.67 |
| Wild strain 2 | 6.67 | Wild strain 8 | 6.67 |
| Wild strain 3 | 6.67 | Wild strain 9 | 6.67 |
| Wild strain 4 | 3.33 | Wild strain 10 | 13.33 |
| Wild strain 5 | 13.33 | Wild strain 11 | 13.33 |
| Wild strain 6 | 13.33 | Wild strain 12 | 6.67 |

### Example 3

### Healing accelerating action on acetic acid ulcer of rat Since it was confirmed that PR-39 exhibits a good antimicrobial activity to H. pylori, as described in the above examples, the healing accelerating effect to an actual ulcer was examined as an in vivo test.

### Method:

15 Sprague Dawle rats each having a weight of about 220 g were used, and a gastric ulcer was experimentally formed in each of the rats. A gastric ulcer was formed by exposing the gastric anterior wall of each of the rats by ventrotomy under etherization, and injecting 0.03 ml of 20% acetic acid into the submucous layer in the boundary between the fundus glands and the pyloric glands of the gastric anterior wall by a Hamilton microsyringe in accordance with the method of Okabe et al., closing the stomach, and then feeding for 7 days in a normal manner.

On the seventh day, five rats were sacrificially killed, and the stomachs were extracted therefrom. The residual 10 rats were divided into two groups consisting of 5 rats each and used as a control group administered with physiologic saline and an experimental group administered with PR-39.

The rats in the control group and the experimental group were intraperitoneally administered with 1 ml of physiologic saline and 0.8 mg/ml of PR-39, respectively, once a day. After administration of physiologic saline or PR-39 for 7 days, the stomach was extracted from each of the rats on the day after final administration (14 days after acetic acid administration).

In each of the extracted stomachs, the pyloric ring and the esophagogastric junction were clamped, and 10 ml of 70% alcohol was injected into the stomach, followed by fixing. The stomach was cut along the curvatura ventriculi major to expose the gastric mucosal surface, and the gastric ulcer formed was visually observed to measure the surface area thereof.

Successively, a central portion of the gastric ulcer was cut out in parallel to the curvatura ventriculi minor, embedded in paraffin, and then stained with HE to histologically examine the formed gastric ulcer.

The examination terms included the length of each of the gastric ulcer, and the thickness of the granulation of the ulcer bottom thereof.

### Results:

As a result of visual observation of a typical gastric mucosa, a deep gastric ulcer having a clear boundary was observed 7 days after administration of acetic acid. 14 days after administration of acetic acid, in the control group administered with physiologic saline, the size and depth of each of ulcers were smaller than those 7 days after administration of acetic acid, thereby confirming that the ulcers are in a healing stage. However, a tissue defect was still apparently observed. On the other hand, in the group administered with PR-39, ulcer scar accompanied with concentration of the mucosal folds of gallbladder was observed, and it was thus indicated that healing is promoted as compared with the control group.

Table 5 shows the results of measurement of the ulcer surface area, and Table 6 shows the results of histological observation.

**Table 5**

| Healing effect of PR-39 on acetic acid ulcer (rat) | | |
|---|---|---|
| | Number of animals | Ulcer surface area (mm2; average±SEM) |
| 7 days after | 5 | 24.5 ± 2.5 |
| 14 days after Administration of physiologic saline | 5 | 8.94 ± 1.65 |
| Administration of PR-39 | 5 | 2.82 ± 1.96 |

**Table 6**

| Results of histological observation of PR-39 for healing of acetic acid ulcer | | | |
|---|---|---|---|
| | Number of animals | Diameter of ulcer (mm) | Thickness of granulation tissue (mm) |
| 7 days after | 5 | 4.86 ± 0.33 | 2.20 ± 0.16 |
| 14 days after Administration of physiologic saline | 5 | 3.58 ± 0.35 | 2.30 ± 0.58 |
| Administration of PR-39 | 5 | 1.58 ± 0.49 | 1.39 ± 0.59 |

The results shown in Table 5 indicate that in the control group administered with physiologic saline, the ulcer area after 14 days after administration of acetic acid is significantly smaller than that 7 days after administration of acetic acid, and thus the days from the 7th to 14th day are in a healing stage. When PR-39 is administered every day in the healing stage, the ulcer area is significantly decreased, as compared with the control group.

Judging from the results, it is understood that exogenous administration of PR-39 accelerates healing of acetic acid ulcers of rats.

In the results of histological observation, the results shown in Table 6 indicate that like in the visual observation, the length of an ulcer in the PR-39 administration group is significantly decreased.

It has been reported that in an acetic acid ulcer model, granulation tissue appears at the ulcer bottom, but the maturation value of granulation can be evaluated from the thickness of the granulation tissue.

From this viewpoint, the fact that the thickness of granulation at the ulcer bottom is significantly decreased by administering PR-39 indicates that PR-39 accelerates maturation of the granulation tissue.

Judging from the above results, it is found that PR-39 matures the granulation at the ulcer bottom and accelerates healing of an ulcer.

### Industrial Applicability

Antimicrobial peptides have the function to eliminate H. pylori and heal an ulcer, and are effective for healing and recurrence of a peptic ulcer such as a chronic ulcer or a gastric ulcer, and useful as a therapeutic agent for diseases of the upper digestive tract, such as gastric cancer lymphoma or the like. Particularly, endogenous antimicrobial peptides such as PR-39 and the like are excellent medicines for preventing or healing peptic ulcers, and significantly contribute to medical care.

## Claims

1. An anti Helicobacter pylori material containing an antimicrobial peptide as an active component.

2. An anti Helicobacter pylor material according to Claim 1, wherein the antimicrobial peptide belongs to the cathelin family or defensin family.

3. An anti Helicobacter pylori material according to Claim 1, wherein the antimicobial peptide is PR-39 peptide.

4. An antiulcer agent containing an antimicrobial peptide as an active component.

5. An antiulcer agent according to Claim 4, wherein the antimicrobial peptide belongs to the cathelin family or defensin family.

6. An antiulcer agent according to Claim 4, wherein the antimicobial peptide is PR-39 peptide as an active component.

7. A wound healing agent containing an antimicrobial peptide as an active component.

8. A wound healing agent according to Claim 7, wherein the antimicrobial peptide belongs to the cathelin family or defensin family.

9. A wound healing agent according to Claim 7, wherein the antimicobial peptide is PR-39 peptide.

10. A method of eliminating H. pylori using an antimicrobial peptide.

11. A bacteria eliminating method according to Claim 10, wherein the antimicrobial peptide belongs to the cathelin family or defensin family.

12. A bacteria eliminating method according to Claim 10, wherein the antimicobial peptide is PR-39 peptide.

13. A method of healing a patient having an ulcer using an antimicrobial peptide.

14. A healing method according to Claim 13, wherein the antimicrobial peptide belongs to the cathelin family or defensin family.

15. A healing method according to Claim 13, wherein the antimicobial peptide is PR-39 peptide.

16. A method of healing a patient having a wound using an antimicrobial peptide as an active component.

17. A healing method according to Claim 16, wherein the antimicrobial peptide belongs to the cathelin family or defensin family.

18. A healing method according to Claim 16, wherein the antimicobial peptide is PR-39 peptide.
